# EUROPEAN PATENT SPECIFICATION

(11) **EP 0 777 487 B1**
(45) Date of publication and mention of the grant of the patent: **06.02.2002**
(21) Application number: 95926813.7
(22) Date of filing: 02.08.1995
(51) Int. Cl.: A61K 31/715

(54) **TREATMENT OF STROKE ASSOCIATED WITH MACROPHAGE INFILTRATION**
BEHANDLUNG VON SCLAGANFALL MIT EINER INFILTRATION VON MAKROPHAGEN ASSOZIIERT
TRAITEMENT DE L' APOPLEXIE ASSOCIEE A DES INFILTRATIONS DE MACROPHAGES

(30) Priority: 24.08.1994 CA 2130762
(43) Date of publication of application: 11.06.1997
(73) Proprietor: JAGOTEC AG, 4132 Muttenz (CH)
(72) Inventor: TURLEY, Eva, Anne, Winnipeg, Manitoba R3B 0R4 (CA); ASCULAI, Samuel, Simon, Toronto, Ontario M6M 2B6 (CA)
(74) Representative: Bassil, Nicholas Charles
(86) International application number: CA9500467
(87) International publication number: WO9605845

(56) References cited:
- EP-A- 0 197 718
- EP-A- 0 557 118
- WO-A-91/04058
- WO-A-93/16732
- US-A- 4 725 585
- J. CLIN. INVEST., vol. 88, November 1991 pages 1622-1628, A. WALDENSTRÖM ET AL. 'Accumulation of hyaluronan and tissue edema in experimental myocardial infarction'
- TEX. REP. BIOL. MED., vol. 39, 1979 pages 339-355, M.R. SHETLAR ET AL. 'Healing of myocardial infarction in animal models'

## Description

### FIELD OF INVENTION

This invention relates to the treatment of diseases and conditions characterized by leukocyte [e.g. white blood cells] infiltration into the area damaged by the disease or condition for example in oxygen and/or glucose deprived tissue in the human body. This invention finds application in the treatment of stroke.

### BACKGROUND OF THE INVENTION

When tissue (and the individual cells) are deprived of oxygen and/or glucose, the cells and consequently the tissue made up by the cells are damaged. As a result, among other responses, an inflammatory response (reaction) is set up in the area (site) of the damage. This inflammatory response includes, among other responses, the migration of inflammatory cells (for example macrophages, neutrophils, and other white blood cells) to the site of the damage.

For example, during a stroke, whatever the cause, the blood supply in the blood vessels is impaired and diminished. The consequences include deprivation of oxygen and glucose resulting in, at the very least, damage to the deprived areas.

This damage sets, among other responses, an inflammatory response in the area damaged with the consequent migration of inflammatory cells (macrophages, neutrophils and other white blood cells). Because of the damage (trauma) to the site, prostaglandin synthesis also increases.

Cerebral deprivation of oxygen and glucose in premature birth follows the same scenario - a deprivation of oxygen to the brain of the infant which sets up an inflammatory response (migration of inflammatory cells (for example macrophages, neutrophiles and other white blood cells).

Pharmaceutical compositions containing hyaluronic acid and a therapeutically active agent are described in WO 91/04058. Uses of such compositions in the treatment of certain medical conditions are also described. EP-A-0357118 describes the use of pharmaceutical composition comprising hyaluronic acid to repair ischemia reperfusion damage in tissue and US-A-4725585 describes administration of hyaluronic acid compositions to stimulate a patient's immune system is disease conditions. The accumulation of hyaluronan and development of tissue edema in rats which had experimentally induced myocardial infarctions has also been reported, suggesting the use of hyaluronidase to treat damage due to myocardial ischemia. None of these documents disclose or suggest a means for treatment of the consequences of stroke.

It is therefore an object of this invention to provide pharmaceutical compositions (for example, injectibles (sterile)), methods of treatment, and new uses for known chemicals which reduce the damage caused to the tissue and cells, resulting from a stroke, characterized by an inflammatory response for example by macrophage, neutrophil or other white blood- cell infiltration or migration into the area of the damage.

Further and other objects of the invention will be realized by those skilled in the art from the following summary of invention and detailed description of embodiments thereof.

### SUMMARY OF THE INVENTION

According to a first aspect of the invention there is provided the use of hyaluronic acid and/or pharmaceutically acceptable salts thereof in the manufacture of a medicament for the treatment of damage caused to tissue and cells resulting from stroke characterised by macrophage, neutrophil or other white blood cell infiltration into the area of damage.

According to this aspect of the invention, when tissue and thus cells are damaged for example by being deprived of glucose and/or oxygen, the activity of inflammatory cells (for example macrophages, neutrophils and other white blood cells) will be modulated (for example their migration into the area (to the site) of damage will be diminished), by the administration of an effective amount of hyaluronic acid and/or salts thereof (for example sodium hyaluronate) at the time the area is being damaged (as for example at the time of a stroke which is characterized by macrophage, neutrophile or other white blood cell infiltration into the area of the tissue damaged by the condition and/or disease or a short time thereafter. While Applicant should not be limited to the following mechanism of action of the invention, Applicant believes that the administration of the hyaluronic acid and/or salts thereof (e.g. sodium salt) blocks, for example by binding with, the hyaluronic acid (HA) receptors of the inflammatory cells (for example macrophages, neutrophiles and other white blood cells), thus blocking their migration into the area of the damaged tissue.

The preferred form of hyaluronic acid is sodium hyaluronate having a molecular weight less than 750,000 daltons for example 10,000 - 300,000 daltons.

To supplement this inhibition (blockage) of the inflammatory response, (which inflammatory response causes the migration or infiltration of the macrophages, neutrophiles or other white blood cells into the damaged area), NSAIDS may also be given with the form of hyaluronic acid which blocks (for example binds with) the HA receptors. Thus inhibition of prostaglandin synthesis can be achieved.

The hyaluronic acid and/or salts (preferably sodium hyaluronate having a molecular weight less than 750,000 daltons) may be utilized at varying doses (depending upon the method of administration) from 1-10 mg/kg body weight to 15 - 20 mg/kg of body weight or more, for example a dose in excess of 25 mg/kg and more to over 3000 mg/70 kg person. In adult human (and adult rats) excess amounts of the form of hyaluronic acid are tolerated, however in rat neonates, excess can and does cause damage.

Thus and according to another aspect of the invention when an NSAID for example indomethacin (dissolved in n-methyl glucamine (nmg)) or other NSAID is administered with greater than 200 mg hyaluronic acid per 70 kg person with 1 - 2 mg/kg body weight of the NSAID (in one instance indomethacin and NMG), no major toxic side effects occur such as gastro-intestinal distress, neurological abnormalities, depression, etc., even at elevated amounts of indomethacin (if necessary). If the amount of hyaluronic acid is decreased below that amount, the usual side effects of using an NSAID may begin to reoccur. The same can be said with other therapeutic agents, no major toxic sid effects occur with the administration of greater than 200 mg hyaluronic acid (e.g., sodium hyaluronate per 70 kg-person.

Preferably (and on the basis of tests performed) each preferred dosage amount of the preferred form of hyaluronic acid, sodium hyaluronate, should be in the order of about 10-25 mg/kg body weight, for example in the order of about 1800 mg/70 kg person if administered subcutaneously for example in the back. Intravenous dosing could employ smaller (lesser) dose amounts of the form of hyaluronic acid. Preferred amounts are 10-20 mg./kg of body weight of a human.

Presently testing in neonate rats reveals that suitable dosage amounts will establish a concentration volume of the chosen form of hyaluronic acid (for example sodium hyaluronate) of about 3 mg/ml of blood in an adult human. When administered, an amount of between about 10mg of, for example, sodium hyaluronate/kg and about 25mg of, for example, sodium hyaluronate/kg of body weight of an adult human. More recent tests with rats neonates, achieved levels of hyaluronic acid (12 hours after administration subcutaneously in the neonates) at 10 mg/kg in their blood. These amounts can be adjusted up or down as would be deemed preferable in the circumstances.

The administration preferably starts at the time of the stroke occurring or shortly thereafter (within 24 hours) and continues until such time as not required. Preferably the level achieved is maintained in the blood. For example a level of 15 mg/kg of body weight is established in the blood, by initial intravenous administration. Then that level is maintained by for example subcutaneous administration (for example, by subcutaneous injection).

NSAIDS may be given at the same time in effective amounts (e.g. 1-2 mg/kg of body weight in the case of indomethacin.

Drugs for the treating of a stroke may also be administered such as a clot dissolving drug. Clot dissolving drugs comprise TPA, Streptokinase (proteolytic product), Urokinase and the like. Other available drugs are the NSAID acetylsalicylic acid (aspirin), beta blockers, heparin, a plasminogen activator, and other suitable drugs.

In addition to the NSAIDS, other drugs (where a stroke is or has occurred) may be administered with the form of hyaluronic acid (for example sodium hyaluronate having a molecular weight less than 750,000 daltons for example 300,000 daltons) with or without the NSAIDS. These drugs would be in their expected amounts. These drugs may be for example the same as above and may also comprise anti-platelet drugs for example those which alter the platelet function (prevent agreregation) and prevent thrombis formation (clots).

One form of hyaluronic acid and/or salts thereof (for example sodium salt) and homologues, analogues, derivatives, complexes, esters, fragments, and sub units of hyaluronic acid, preferably hyaluronic acid and salts and thereof suitable for use with Applicant's invention is a fraction supplied by Sterivet Laboratories Limited. One such fraction is a 15 ml vial of Sodium hyaluronate 20 mg/ml (300 mg/vial - Lot 2F3). The sodium hyaluronate fraction is a 2% solution with a mean average molecular weight of about 225,000. The fraction also contains water q.s. which is triple distilled and sterile in accordance with the U.S.P. for injection formulations. The vials of hyaluronic acid and/or salts thereof may be carried in a Type I borosilicate glass vial closed by a butyl stopper which does not react with the contents of the vial.

The fraction of hyaluronic acid and/or salts thereof (for example sodium salt) may comprise the following characteristics:
a purified, substantially pyrogen-free fraction of hyaluronic acid obtained from a natural source having at least one characteristic selected from the group consisting of the following:
   (i) a molecular weight within the range of 150,000-225,000;
   (ii) less than about 1.25% sulphated mucopolysaccharides on a total weight basis;
   (iii) less than about 0.6% protein on a total weight basis;
   (iv) less than about 150 ppm iron on a total weight basis;
   (v) less than about 15 ppm lead on a total weight basis;
   (vi) less than 0.0025% glucosamine;
   (vii) less than 0.025% glucoronic acid;
   (viii) less than 0.025% N-acetylglucosamine;
   (ix) less than 0.0025% amino acids;
   (x) a UV extinction coefficient at 257 nm of less than about 0.275;
   (xi) a UV extinction coefficient at 280 nm of less than about 0.275;
   (xii) a pH within the range of 7.3-7.9.

Preferably the hyaluronic acid is mixed with water and the fraction of hyaluronic acid fraction has a mean average molecular weight within the range of 150,000-225,000. More preferably the fraction of hyaluronic acid comprises at least one characteristic selected from the group consisting of the following characteristics:
(i) less than about 1% sulphated mucopolysaccharides on a total weight basis;
(ii) less than about 0.4% protein on a total weight basis;
(iii) less than about 100 ppm iron on a total weight basis;
(iv) less than about 10 ppm lead on a total weight basis;
(v) less than 0.00166% glucosamine;
(vi) less than 0.0166% glucuronic acid;
(vii) less than 0.0166% N-acetylglucosamine;
(viii) less than 0.00166% amino acids;
(ix) a UV extinction coefficient at 257 nm of less than about 0.23;
(x) a UV extinction coefficient at 280 nm of less than 0.19; and
(xi) a pH within the rant of 7.5-7.7.

Other forms of hyaluronic acid and/or its salts, and homologues, derivatives, complexes, esters, fragments and sub units of hyaluronic acid may be chosen from other suppliers, for example those described in the prior art documents previously referred to. In addition Applicants have propose sodium hyaluronate produced and supplied by LifeCore™ Biomedical, Inc. having the following specifications

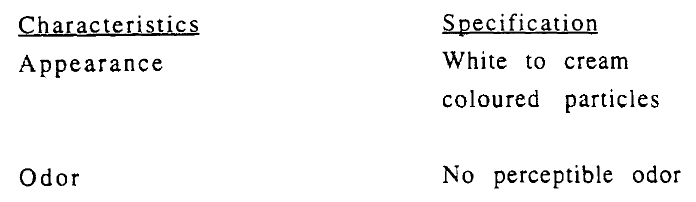

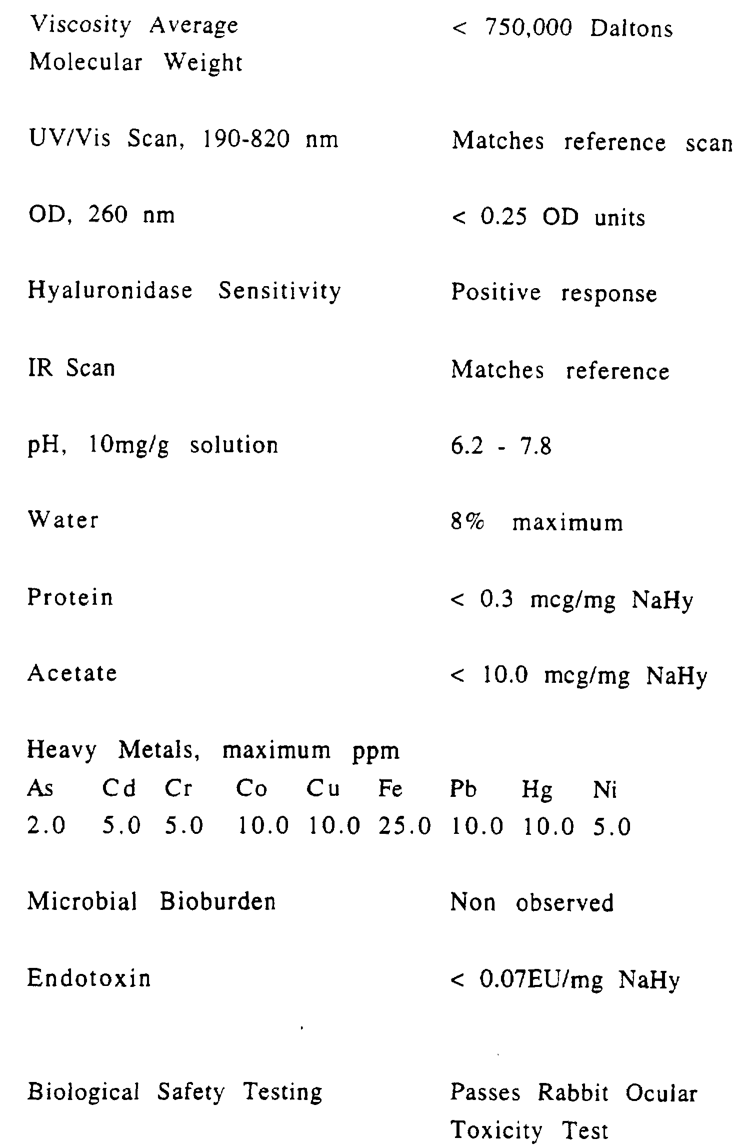

The administration may take place subcutaneously, intravenously or by injection.

### DETAILED DESCRIPTION OF EMBODIMENTS

The invention will now be illustrated with respect to the following example.

### Experiment 1

Seven day old Fischer rat neonates were injected subcutaneously with sodium hyaluronate (MW 300,000 daltons) in the back 1/2 hour before the operation with 0.6 mg of sodium hyaluronate /60ul/animal. Animals were then injected once a day for seven days after the operation and euthenized 14 days after the operation. The right carotid artery was ligated for 1 hour (induced stroke). The animals were then placed in incubators containing 8% oxygen. (The left side was not tied off and provided a suitable control.) Brain damage was readily produced in control animals by day 4 and 7 as determined by nissl staining (for nerves) and for gliosis including increased staining for GFAP, connexin 43, and macrophages (ED-1 epitope), As well increased staining for hyaluronan receptors was observed with CD44 increased in macrophages and astrocytes while RHAMM was increased in neuronal cells and subsets of macrophages. As well damage was observed morphologically where neuronal loss was evident and the right half of the brain had collapsed. Animals treated with HA for 7 days were euthanized at 14 days (as were controls run with these experiments) and none of the above parameters were positive. That is there is no evidence of brain collapse, neuronal loss, macrophage influx or increase in the expression of gliotic proteins or hyaluronan receptors. The hyaluronan treated animal's brains appeared morphologically normal, although functional test for neuronal activity have not yet been done. In the absence of any obvious morphological changes, extensive functional damage would not be expected.

The dose used for each animal was 0.6mg of sodium hyaluronate (Molecular Weight 300,000 daltons) per 23g animal by subcutaneous administration so for a human of 70 kg this would mean a 1.8g subcutaneous dose/person. An intravenous dose would be smaller.
There are approximately 85 cc blood/kg in humans. Therefore an average adult would have about 6000 cc (70 kg person). Thus the concentration achieved is in the order of 0.3 mg of sodium hyaluronate/l ml or cc of blood. Administration to achieve this concentration is in the order of 25 mg/kg of body weight for humans.

### Experiment 2

Experiment 2 repeated Experiment 1 only the right carotid artery was ligated for 3 hours (2 hours longer than the 1 hour specified in Experiment 1) to accrue more brain necrosis. In Experiment 2 the same amount of sodium hyaluronate (HA) (0.6 mg of sodium hyaluronate) was administered to each neonate (regardless of the actual weight of the neonates) and each animal received an injection of sodium hyaluronate subcutaneously at the time of the operation.

Twelve hours after subcutaneous administration, blood levels of 15-20 mg/kg of HA are obtained. Continued analysis of blood levels indicates that HA levels remain at 15 mg/kg for 24 hours.

After the operation, we continued to inject HA in the same dosage amounts every 24 hours for 7 days. At not time during the 7 days did the HA levels drop below 15 mg/kg.

The brains of the animals (including controls) were examined at 2 weeks.

Of the three animals injected with HA, the brains were in the same condition as the brains of the neonates administered with HA in Experiment 1. The one control suffered excessive brain damage. As a result of these tests, we have concluded that dose amounts of as low as 1 mg/kg of body weight of the animal will be therapeutic (e.g. for blocking the infiltration of macrophages, neutrophiles and other white blood cells into the area (to the site) of the stroke. Dose amounts of 10 mg or more of HA/kg of the animal (human) are preferred for example 10-20 mg/kg of body weight.

### Experiment 3

While humans and adult rats are able to tolerate excesses of sodium hyaluronate (HA), rat neonates are not as tolerant. Thus, in Experiment 3, where the rate neonates were smaller, dosage amounts of 25 mg/kg of HA, administered in Experiment 3, resulted in damage to the brains of the neonates.

### Comparative Experiment 1

Four rats were each exposed to isoproteranol to induce myocardial infarction (heart attack) in each. The administration of isoproteranol for inducing an infarct is a commonly known technique as would be understood by persons skilled in the act and is not elaborated herein. Each of two of the rats was immediately after the infarct injected with sodium hyaluronate (Molecular Weight 300,000 daltons) (HA) in the amount of 15 mg/kg. The subcutaneous injections continued for seven days (one subcutaneous injection each day). Twelve hours after the initial injection, the blood levels of HA were 10 mg/kgm body weight in the blood system of each rat.

The other two rats were immediately after the induction of the infarct each injected subcutaneously with saline (as a control). The subcutaneous injections continued for seven days, one injection per day. The rats were then sacrificed. In the saline-treated animals, heart tissue was necrotic with massive amounts of accumulated white cells. In the HA-treated rats, no damage was observed in the heart tissue and no white cells were apparent (as determined by EO-1 staining of frozen sections).

As a result, we have concluded that dosage amounts of 10 - 25 mg/kg of body weight administered to humans is appropriate. While lesser amounts can still be therapeutic, they will not give optimal results. Optimal results are the goal in the treatment of each of a stroke and myocardial infarction.

Preferably the chosen dosage amount of the HA is initially given intravenously to establish the desired levels of HA in the blood. Thereafter, these levels are maintained for example by administration subcutaneously (subcutaneous injection). Preferred blood levels appear to be starting in the order of 10 mg/kg.

As many changes can be made to the preferred embodiments of the invention without departing from the scope of the invention, it is intended that all material contained herein be interpreted as illustrative of the invention and not in a limiting sense.

## Claims

1. The use of hyaluronic acid and/or pharmaceutically acceptable salts thereof in the manufacture of a medicament for the treatment of damage caused to tissue and cells resulting from stroke **characterised by** macrophage, neutrophil or other white blood cell infiltration into the area of the damage.

2. A use as claimed in claim 1, in which the medicament further comprises an NSAID, an anti-stroke drug, a clot dissolving drug, a beta blocker, acetylsalicylic acid, streptokinase, anti-platelet drugs, heparin or a plasminogen activator or combinations thereof.

3. A use as claimed in claim 1 or claim 2, in which the hyaluronic acid or salt thereof is sodium hyaluronate having a molecular weight less than 750,000 daltons.

4. A use as claimed in claim 1 or claim 2, in which the hyaluronic acid or salt thereof is sodium hyaluronate having a molecular weight less than 300,000 daltons.

5. A use as claimed in any one of claims 1 to 4 in which the medicament is suitable for intravenous or subcutaneous injection.

## Patentansprüche

1. Verwendung von Hyaluronsäure und/oder pharmazeutisch annehmbaren Salzen davon in der Herstellung eines Medikamentes zur Behandlung von Gewebe- und Zellschädigungen, die durch einen Schlaganfall hervorgerufen wurden, **gekennzeichnet durch** Infiltration von Makrophagen, Neutrophilen oder anderen weißen Blutkörperchen in den geschädigten Bereich.

2. Verwendung nach Anspruch 1, wobei das Medikament weiters ein NSAID, ein Anti-Schlaganfall Arzneimittel, ein Klumpen-auflösendes Arzneimittel, einen Beta-Blocker, Acetylsalicylsäure, Streptokinase, ein Anti-Thrombozyten Arzneimittel, Heparin oder einen Plasminogenaktivator oder Kombinationen davon enthält.

3. Verwendung nach Anspruch 1 oder 2, wobei die Hyaluronsäure oder das Salz davon Natriumhyaluronat mit einem Molekulargewicht von weniger als 750 000 Dalton ist.

4. Verwendung nach Anspruch 1 oder 2, wobei die Hyaluronsäure oder das Salz davon Natriumhyaluronat mit einem Molekulargewicht von weniger als 300 000 Dalton ist.

5. Verwendung nach einem der Ansprüche 1 bis 4, wobei das Medikament für intravenöse oder subkutane Injektionen geeignet ist.

## Revendications

1. Utilisation de l'acide hyaluronique et/ou de sels pharmaceutiquement acceptables de celui-ci dans la production d'un médicament pour le traitement d'une lésion causée à un tissu et à des cellules résultant d'une attaque **caractérisée par** une infiltration de macrophages, de polynucléaires neutrophiles ou d'autres globules blancs dans la zone de la lésion.

2. Utilisation selon la revendication 1, dans laquelle le médicament contient en outre un médicament AINS, un médicament anti-attaque, un médicament qui dissout les caillots, un bêta-bloquant, de l'acide acétylsalicylique, de la streptokinase, des anti-agrégants plaquettaires, de l'héparine ou un activateur de plasminogène ou des combinaisons de ceux-ci.

3. Utilisation selon la revendication 1 ou la revendication 2, dans laquelle l'acide hyaluronique ou un sel de celui-ci est l'hyaluronate de sodium ayant un poids moléculaire inférieur à 750 000 daltons.

4. Utilisation selon la revendication 1 ou la revendication 2, dans laquelle l'acide hyaluronique ou un sel de celui-ci est l'hyaluronate de sodium ayant un poids moléculaire inférieur à 300 000 daltons.

5. Utilisation selon l'une quelconque des revendications 1 à 4, dans laquelle le médicament est approprié pour une injection intraveineuse ou sous-cutanée.
